Europäisches Patentamt

European Patent Office

Office européen des brevets

(19)

(11) Veröffentlichungsnummer : **0 410 279 B1**

(12) # EUROPÄISCHE PATENTSCHRIFT

(45) Veröffentlichungstag der Patentschrift :
**29.09.93 Patentblatt 93/39**

(51) Int. Cl.$^5$ : **C07C 47/02, C07C 45/50**

(21) Anmeldenummer : **90113714.1**

(22) Anmeldetag : **18.07.90**

(54) **Hydroformylierungsverfahren.**

(30) Priorität : **26.07.89 DE 3924720**

(43) Veröffentlichungstag der Anmeldung :
**30.01.91 Patentblatt 91/05**

(45) Bekanntmachung des Hinweises auf die
Patenterteilung :
**29.09.93 Patentblatt 93/39**

(84) Benannte Vertragsstaaten :
**BE DE ES FR GB IT NL**

(56) Entgegenhaltungen :
**EP-A- 0 114 611**
**EP-A- 0 179 004**
**EP-A- 0 254 180**
**DE-B- 1 793 069**
**US-A- 4 258 215**
**US-A- 4 711 968**

(73) Patentinhaber : **BASF Aktiengesellschaft**
**Carl-Bosch-Strasse 38**
**D-67063 Ludwigshafen (DE)**

(72) Erfinder : **Koeffer, Dieter,Dr.**
**Zinkgraefstrasse 1**
**D-6940 Weinheim (DE)**
Erfinder : **Bertleff, Werner, Dr.**
**Franz-Marc-Strasse 12**
**D-6806 Viernheim (DE)**
Erfinder : **Roeper, Michael, Dr.**
**Albert-Schweitzer-Strasse 10**
**D-6706 Wachenheim (DE)**

EP 0 410 279 B1

## Beschreibung

Die vorliegende Erfindung betrifft ein Verfahren zur Hydroformylierung olefinischer Verbindungen in einem organischen Reaktionsmedium und mit Hilfe von im Reaktionsmedium löslichen Rhodiumkomplexkatalysatoren.

Die Hydroformylierungsreaktion olefinischer Verbindungen mittels homogen im organischen Reaktionsmedium gelöster Rhodium-Komplexkatalysatoren ist allgemein bekannt, beispielsweise aus Chem. Eng. Dec. 1977, S. 110 ff. oder aus DE-A 33 01 591, DE-A 17 93 069 und DE-A 11 86 455. Zur Stabilisierung des Katalysators und zur Verbesserung der Selektivität für n-Aldehyde werden Phosphin- oder Phosphit-Liganden dem Reaktionsmedium zugesetzt, und zwar in einem hohen molaren Überschuß bezüglich des als Katalysators eingesetzten Rhodiums.

Im Zuge der industriellen Hydroformylierungsverfahren wird der Rhodiumkatalysator allmählich desaktiviert. Als Ursache dieser Desaktivierung gelten zum einem Katalysatorgifte, welche mit den Einsatzstoffen ins Reaktionsmedium eingetragen werden, zum anderen Abbaureaktionen der Phosphor-Liganden. Als Katalysatorgifte sind insbesondere Schwefelverbindungen wie Schwefelwasserstoff und Carbonylsulfid bekannt, weshalb diese Verbindungen aus den Strömen der Einsatzstoffe des Hydroformylierungsverfahrens möglichst vollständig entfernt werden müssen (vgl. B. Cornils, in J. Falbe, New Syntheses with Carbon Monoxide, insbesondere S. 73-77, Springer, Berlin 1980).

Von den Abbaureaktionen der Phosphorliganden weiß man, daß z.B. aus Triphenylphosphin bei der Propen-Hydroformylierung zunächst Alkyldiphenyl- und Dialkylphenylphosphine wie Propyldiphenyl-, Butyldiphenyl- oder Dipropylphenylphosphin gebildet werden, welche dann weiter abgebaut werden (vgl. B. Cornils in J. Falbe, New Syntheses with Carbon Monoxide, (1980), S. 73-77).

Zwar können gemäß US-A 4 711 968 auch schwefelhaltige Cracker-Destillate mit Hilfe von Kobalt- oder Rhodium-Komplex-Katalysatoren hydroformyliert werden, doch handelt es sich bei den in diesen Destillaten enthaltenen Schwefelverbindungen vorwiegend um Thiophene und Benzothiophene, welche auf die Hydroformylierungskatalysatoren nicht inhibierend wirken und nur in geringem Maße um Thiole, deren nachteilige Wirkung auf die Katalysatoren durch eine Reihe von Maßnahmen, beispielsweise die Anwendung eines höheren Kohlenmonoxid-Partialdruckes zu verhindern versucht wird.

Kalck et al. beschreiben die Hydroformylierung mit t-Butylthiolat-verbrückten, zweikernigen Rhodiumkomplexen mit Phosphit- (J. Chem. Soc. Chem. Com. 1983, S. 510-511) oder Phosphinliganden (J. Organometal. Chem. 302, C17-C20 (1986)). Mit t-Butylthiolat als Thiol-Spezies verbrückte, zweikernige, wasserlösliche Rhodiumkomplexe mit Triarylphosphin-trisulfonat-Liganden werden auch in EP-A 179 004 bevorzugt eingesetzt, obwohl darin auch die Herstellung Arylthiolat-verbrückter, wasserlöslicher Rhodium-Triarylphosphin-trisulfonat-Komplexe beschrieben ist. Diese t-Butylthiolatverbrückten Rhodiumkomplex-Katalysatoren zeichnen sich bei Laborversuchen durch eine höhere Reaktivität und eine verbesserte Selektivität gegenüber den herkömmlichen Rhodiumkomplex-Katalysatoren aus. Beim Einsatz dieser t-Butylthiolat-verbrückten Rhodiumkomplexe in kontinuierlich betriebenen Versuchsanlagen zur Hydroformylierung wurde jedoch festgestellt, daß die t-Butylthiolat-Komplexe weitgehend wirkungslos bleiben und daß t-Butylthiol nach und nach, zusammen mit den Hydroformylierungsprodukten aus dem Reaktionsmedium ausgetragen wird.

Es bestand nun die Aufgabe ein Verfahren zur Hydroformylierung olefinisch ungesättigter Verbindungen mit Hilfe wasserunlöslicher Rhodium-Katalysatoren zu finden, bei dem die Desaktivierung des Rhodium-Katalysators infolge der Abbaureaktionen der Phosphin- oder Phosphit-Liganden verhindert und die Langzeit-Selektivität und Stabilität des Katalysators verbessert wird.

Dementsprechend wurde ein Verfahren zur Hydroformylierung olefinischer Verbindungen in einem organischen Reaktionsmedium und mit Hilfe von im Reaktionsmedium löslichen Rhodiumkomplex-Katalysatoren gefunden, das dadurch gekennzeichnet ist, daß man die Umsetzung in Gegenwart wasserunlöslicher Rhodiumkomplexkatalysatoren mit Triorganophosphin- oder Triorganophosphit-Liganden und in Gegenwart von Arylthiolen der allgemeinen Formel I

$$Ar\text{-}SH \qquad I$$

in der Ar eine substituierte oder unsubstituierte Arylgruppe ist, durchfuhrt und das Arylthiol I in einem Molverhältnis von 0,1 bis 20 pro mol Rhodium verwendet.

Als Arylthiole I können im erfindungsgemäßen Verfahren unsubstituierte Arylthiole wie Thiophenol, Naphthalinthiol-1, Naphthalinthiol-2, Anthracenthiol-1 oder Phenanthrenthiol-1 oder mit 1 bis 3 $C_1$-$C_4$-Alkyl- und/oder Alkoxygruppen substituierte Thiole wie 2-Methylthiophenol, 3-Methylthiophenol, 4-Methylthiophenol, 2,4-Dimethylthiophenol, 3,4,5-Trimethylthiophenol, 4-Ethylthiophenol, 4-n-Propylthiophenol, 4-Isopropylthiophenol, 4-t-Butylthiophenol, 4-Methoxythiophenol oder 4-t-Butoxythiophenol verwendet werden. Diese Verbindungen sind im Handel erhältlich oder können auf einfache Weise synthetisiert werden, beispielsweise nach den Methoden von Houben-Weyl, Methoden der Organischen Chemie, Band IX, Seiten 7 - 42, Thieme, Stuttgart 1955. Im allgemeinen werden unsubstituierte Arylthiole verwendet. Es versteht sich von selbst, daß an-

2

stelle dieser Arylthiole auch Verbindungen zur Reaktionsmischung hinzugefügt werden, aus denen sich unter den Bedingungen der Hydroformylierungsreaktion die Arylthiole I bilden können, beispielsweise die entsprechenden Diaryldisulfide.

Die Arylthiole I werden erfindungsgemäß in einem Molverhältnis von 0,1 bis 20 mol/mol Rhodium, vorzugsweise in ein Molverhältnis von 1 bis 5 mol/mol Rhodium zur Reaktionsmischung zugefügt.

Als Hydroformylierungskatalysatoren werden im allgemeinen wasserunlösliche Rhodiumkomplexe mit Triorganophosphorverbindungen als Liganden verwendet, wie sie beispielsweise in DE-A 28 02 923 und DE-A 17 93 069 beschrieben sind. Vorzugsweise werden Triarylphosphine oder Alkyldiarylphosphine sowie Trialkyl-, Triaryl-, Dialkylaryl- oder Alkyldiarylphosphite benutzt. Besonders bevorzugte Triorganophosphine sind Triarylphosphine wie Triphenylphosphin und Tritolylphosphin oder Alkyldiarylphosphine wie n-Hexyldiphenylphosphin; von den Triorganophosphiten werden Triphenyl- und Tritolylphosphit bevorzugt eingesetzt.

Die Triorganophosphorverbindungen werden im erfindungsgemäßen Verfahren bezüglich des eingesetzten Rhodiums in einem Molverhältnis von 1 bis 500, vorzugsweise von 1 bis 150 mol/mol Rhodium verwendet.

Zweckmäßigerweise werden die Rhodiumkomplex-Katalysatoren in situ im Hydroformylierungsgemisch aus Rhodiumsalzen, beispielsweise aus Rhodiumacetat oder Rhodiumacetonylacetonat und den gewünschten Triorganophosphor-Liganden erzeugt. Selbstverständlich können auch separat hergestellte Rhodiumkomplexe wie Hydridocarbonyltris(triphenylphosphin)rhodium benutzt werden. Entsprechendes gilt für den erfindungsgemäßen Zusatz der Arylthiole I. Diese werden zweckmäßigerweise wie die Rhodium- und die Triorganophosphor-Komponente des Katalysators zur Hydroformylierungsmischung gegeben.

Die Hydroformylierung von Olefinen wird mit den erfindungsgemäß Arylthiolmodifizierten Rhodiumkatalysatoren bei einem Druck von zweckmäßigerweise 1 bis 300, bevorzugt 1 bis 50 bar, und bei einer Temperatur von im allgemeinen 50 bis 160°C vorgenommen. Die weitere Verfahrensweise beispielsweise die Verwendung von Lösungsmitteln, die Kreisgasfahrweise, die Katalysatorrückführung und die Kohlenmonoxid/Wasserstoff-Konzentrationshältnisse sind Stand der Technik und können beispielsweise der eingangs zitierten Literatur DE-A 28 02 923, DE-A 17 93 069, DE-A 33 01 591 sowie DE-A 11 86 455 entnommen werden.

Nach dem erfindungsgemäßen Verfahren können sowohl $\alpha$-Olefine, wie Ethen, Propen, 1-Buten, 1-Hexen, 1-Octen oder 1-Decen als auch Olefine mit innenständiger Doppelbindung, wie 2-Buten, 2-Hexen oder 3-Hexen, sowie mit unter den Reaktionsbedingungen inerten Substituenten substituierte Olefine, beispielsweise Allylalkohol, Allylacetat, Acrylsäureester, Acrylnitril oder Acroleinacetale, hydroformyliert werden.

Das erfindungsgemäße Verfahren ermöglicht es, den Abbau der Phosphor-Liganden und damit die allmähliche Desaktivierung des Katalysators wirksam zu unterdrücken. Dadurch wird der Verlust an Phosphor-Liganden durch unerwünschte Abbaureaktionen wesentlich verringert und die Katalysatorkosten des Verfahrens gesenkt. Weiterhin ermöglicht es das erfindungsgemäße Verfahren, hohe Selektivitäten für n-Aldehyde zu erzielen, ohne daß ein hoher molarer Überschuß von Phosphin- oder Phosphit-Liganden bezogen auf Rhodium erforderlich ist.

Beispiel 1

In einem Versuchsreaktor wurden stündlich 236 g Propen in Gegenwart von 120 ppm Rhodium, 0,37 Gew.-% Triphenylphosphin (P/Rh : 120 mol/mol), 128 ppm Thiophenol (S/Rh : 1,0 mol/mol) - die Mengenangaben sind auf die Menge des Reaktionsgemisches bezogen - und 1000 g Texanol® (= Isomerengemisch des 2,2,4-Trimethylpentan-1,3-diol-3-monoisobutyrats) als Lösungsmittel bei 105°C und einem Druck von 20 bar hydroformyliert. Dem Reaktor wurden Kohlenmonoxid-Wasserstoff-Gasgemisch, Wasserstoff und Kreisgas in einer Menge zugeführt, daß bei einer konstanten Kreisgasmenge von 480 Nl/h und einer Abgasmenge von 30 Nl/h, der Kohlenmonoxid-Gehalt im Kreisgas 4 bis 5 Vol.-% und der Wasserstoffgehalt des Kreisgases ca. 70 Vol.-% betrug. Der flüssige Reaktoraustrag wurde entspannt und über einen Dünnfilmverdampfer in Butyraldehyd-Destillat und das katalysatorhaltige Sumpfprodukt aufgetrennt. Das katalysatorhaltige Produkt wurde kontinuierlich in den Reaktor zurückgeführt.

Die Butyraldehyd-Ausbeute fiel über 21 Versuchstage von 62,2 % auf 58,4 % ab. Der n-Anteil betrug 84 %.

Nach Ende des Versuches hatte die Reaktionsmischung einen Schwefelgehalt entsprechend einem S/Rh-Molverhältnis von 1,1 mol/mol.

Tabelle 1 zeigt die zeitliche Entwicklung des Ligandenabbaus verglichen mit dem des Vergleichsbeispiels A (Hydroformylierung in Abwesenheit eines Arylthiols).

In den folgenden Tabellen bedeuten die Abkürzungen Ph : Phenyl, Pr : Propyl und Bu : Butyl.

Vergleichsbeispiel A

Der Versuch wurde analog zu Beispiel 1, jedoch ohne den Zusatz von Thiophenol durchgeführt.

Die Butyraldehyd-Ausbeute fiel über 21 Versuchstage von 76,8 % auf 60,9 % ab. Der n-Anteil betrug 80 %. In Tabelle 1 ist die zeitliche Entwicklung des Ligandenabbaus dargestellt.

Beispiel 2 und Vergleichsbeispiele B und C

Analog Beispiel 1, jedoch bei einer Reaktionstemperatur von 120°C wurden stündlich 236 g Propen in Gegenwart von 120 ppm Rhodium, 0,37 Gew.-% Triphenylphosphin und einem Thiol gemäß den Angaben von Tabelle 2 hydroformyliert.

Die Bildungsraten der Phosphin-Abbauprodukte nach einer Versuchszeit von 108 h sind in Tabelle 2 gegenübergestellt.

Beispiele 3 bis 6 und Vergleichsbeispiele D und E

Ein Gemisch aus 60 g Octen-1, 60 g Toluol und 29,6 mg (0,12 mmol) (Acetylacetonato-)dicarbonylrhodium sowie Phosphin und Arylthiol gemäß den Angaben der Tabelle 3 wurden in einem 300 ml-Autoklaven 3 h bei 100°C und 30 bar mit einem Kohlenmonoxid-Wasserstoff-Gasgemisch (CO/$H_2$ : 1 mol/mol) hydroformyliert. Der flüssige Reaktionsaustrag wurde gaschromatographisch analysiert. Die Resultate sind in Tabelle 3 zusammengestellt.

Tabelle 1

| Versuchsdauer | Beispiel 1 | | | Vergl.Bsp. A | | |
|---|---|---|---|---|---|---|
| (h) | 180 | 348 | 516 | 180 | 348 | 516 |
| Phosphin-Abbau-produkte in der Reaktionslösung (mmol/kg): | | | | | | |
| $PPh_2Pr$ | 1,91 | 3,87 | 6,25 | 5,43 | 14,9 | 21,0 |
| $PPh_2Bu$ | 0,04 | 0,10 | 0,17 | 0,26 | 1,94 | 3,37 |
| $PPhR_2$ (R = Pr, Bu) | 0,06 | 0,06 | 0,14 | 0,16 | 1,09 | 2,65 |
| Mittlere Bildungs-rate der Phosphin-Abbauprodukte (mmol/kg x d): | | | | | | |
| $PPh_2Pr$ | | 0,30 | | | 0,98 | |
| $PPh_2Bu$ | | 0,008 | | | 0,16 | |
| $PPhR_2$ (R = Pr, Bu) | | 0,007 | | | 0,12 | |

Tabelle 2

| | Beispiel 2 | Vergleichs- beispiel B | Vergleichs- beispiel C |
|---|---|---|---|
| Thiol (mol/mol Rh) | Thiophenol 2,0 | tert.-Butylthiol 2,0 | – |
| Versuchsdauer (h) | 108 | 132 | 108 |
| Mittlere Bildungs- rate der Phosphin- Abbauprodukte (mmol/kg x d): | | | |
| PPh$_2$Pr | 0,24 | 2,73 | 2,49 |
| PPh$_2$Bu | 0,03 | 0,27 | 0,38 |
| PPhR$_2$ (R = Pr, Bu) | 0,00 | 0,29 | 0,52 |
| S/Rh-Verhältnis in der Reaktionslösung nach Versuchsende (mol/mol) | 2,0 | 1,0 | – |

Tabelle 3

| Beispiel | Phosphin (mmol) | P/Rh (mol/mol) | Arylthiol (mmol) | Ums. (%) | Aldehyd selekt. (%) | n-An- teil (%) |
|---|---|---|---|---|---|---|
| Vgl. D | Triphenylphos- phin (0,6) | 5 | | 98 | 92 | 57 |
| Vgl. E | Triphenylphos- phin (1,2) | 10 | | 99 | 88 | 59 |
| 3 | Triphenylphos- phin (1,2) | 10 | Thiophenol (0,24) | 99 | 89 | 68 |
| 4 | Triphenylphos- phin (1,2) | 10 | Thiophenol (0,60) | 99 | 87 | 73 |
| 5 | Triphenylphos- phin (0,6) | 5 | p-Thiokresol (0,12) | 99 | 87 | 65 |
| 6 | Hexyldiphenyl- phosphin (1,2) | 10 | Thiophenol (0,12) | 99 | 93 | 72 |

**Patentansprüche**

1. Verfahren zur Hydroformylierung olefinischer Verbindungen in einem organischen Reaktionsmedium und mit Hilfe von im Reaktionsmedium löslichen Rhodiumkomplexkatalysatoren, dadurch gekennzeichnet, daß man die Umsetzung in Gegenwart wasserunlöslicher Rhodiumkomplex- katalysatoren mit Triorganophosphin- oder Triorganophosphit-Liganden und in Gegenwart von Arylthiolen der allgemeinen Formel I

$$Ar-SH \qquad I$$

in der Ar eine substituierte oder unsubstituierte Arylgruppe ist, durchführt und das Arylthiol I in einem Molverhältnis von 0,1 bis 20 pro mol Rhodium verwendet.

2. Verfahren nach Anspruch 1, dadurch gekennzeichnet, daß die Gruppe Ar für eine mit 1 bis 3 $C_1$- bis $C_4$- Alkyl- und/oder Alkoxygruppen substituierte oder unsubstituierte Phenyl-, Naphthyl-, Anthryl- oder Phenanthryl-Gruppe steht.

3. Verfahren nach Anspruch 1, dadurch gekennzeichnet, daß das Reaktionsmedium, Rhodiumkomplexkatalysatoren enthält, welche Triphenylphosphin oder Hexyldiphenylphosphin als Liganden enthalten.

## Claims

1. A process for hydroformylating olefinic compounds in an organic reaction medium with the aid of a rhodium complex catalyst which is soluble in the reaction medium, which comprises performing the reaction in the presence of a water-insoluble rhodium complex catalyst having a triorganophosphine or triorganophosphite ligand and also in the presence of an arylthiol of the general formula I

$$Ar-SH \qquad I$$

where Ar is substituted or unsubstituted aryl, and using the arylthiol I in a molar ratio of from 0.1 to 20 per mole of rhodium.

2. A process as claimed in claim 1, wherein Ar is unsubstituted or $C_1$-$C_4$-alkyl- or -alkoxy-monosubstituted, -disubstituted or -trisubstituted phenyl, naphthyl, anthryl or phenanthryl.

3. A process as claimed in claim 1, wherein the reaction medium contains a rhodium complex catalyst which contains triphenylphosphine or hexyldiphenylphosphine as ligand.


## Revendications

1. Procédé d'hydroformylation de composés oléfiniques dans un milieu réactionnel organique et avec l'aide de catalyseurs aux complexes de rhodium et solubles dans le milieu réactionnel, caractérisé en ce que l'on effectue la réaction en présence de catalyseurs aux complexes de rhodium insolubles dans l'eau avec des ligands de triorganophosphine ou de triorganophosphite et en présence d'arylthiols de formule générale I

$$Ar-SH \qquad I$$

dans laquelle Ar est un groupe aryle substitué ou non substitué, et en ce que l'on utilise l'arylthiol I dans un rapport molaire de 0,1 à 20 par mole de rhodium.

2. Procédé selon la revendication 1, caractérisé en ce que le groupe Ar est mis pour un groupe phényle, naphtyle, anthryle ou phénanthryle substitué par 1 à 3 groupes alkyle en $C_1$ à $C_4$ et/ou alcoxy, ou non substitué.

3. Procédé selon la revendication 1, caractérisé en ce que le milieu réactionnel contient des catalyseurs aux complexes de rhodium, qui contiennent en tant que ligands la triphénylphosphine ou l'hexyldiphényl-phosphine.